**Europäisches Patentamt**

**European Patent Office**

**Office européen des brevets**

⑩

(11) Publication number: **0 153 013**
**B1**

## (12) EUROPEAN PATENT SPECIFICATION

(45) Date of publication of patent specification: **05.09.90**

(21) Application number: **85300370.5**

(22) Date of filing: **21.01.85**

(51) Int. Cl.⁵: **A 61 K 47/00, A 61 K 31/715, A 23 L 1/308**

(54) **Oral compositions containing dextran.**

(30) Priority: **01.02.84 GB 8402573**

(43) Date of publication of application:
**28.08.85 Bulletin 85/35**

(45) Publication of the grant of the patent:
**05.09.90 Bulletin 90/36**

(84) Designated Contracting States:
**AT BE CH DE FR GB IT LI LU NL SE**

(56) References cited:
**GB-A- 830 951**
**US-A-4 470 975**

**FOOD SCIENCE AND TECHNOLOGY ABSTRACTS, vol. 71, no. 71065592; & S. YAMAZAKI: "Application of colloidal substances to foods": Food Industry Shokuhin Kogyo, 5 ref. 11(2) 74-77, 1969**

**The file contains technical information submitted after the application was filed and not included in this specification**

(73) Proprietor: **FISONS plc**
**Fison House Princes Street**
**Ipswich Suffolk IP1 1QH (GB)**

(72) Inventor: **Alsop, Ranulph Michael**
**28 Windemere Drive**
**Alderley Edge Cheshire (GB)**
Inventor: **Milner, Jeremiah**
**22 Albert Road**
**Cheltenham Gloucestershire (GB)**

(74) Representative: **Craig, Christopher Bradberry et al**
**Fisons plc 12 Derby Road**
**Loughborough Leicestershire LE11 0BB (GB)**

Courier Press, Leamington Spa, England.

**Description**

This invention relates to a novel use of a specific dextran for the manufacture of medicaments based on the finding that compostions as described below surprisingly do not increase the blood glucose or blood insulin level.

Dextrans of a wide variety of molecular weights have been known for many years and certain dextrans, e.g. those having molecular weights of about 40,000 to 70,000, have found use in medicine for administration, by infusion, as blood plasma expanders. Certain dextrans have also, in some countries, been permitted as food additives and indeed native dextrans are sometimes present in small quantities in food grade sucrose. Blood et al Proc Soc Exp Biol Med (1952) *81,* 501 have shown that certain dextrans when ingested by man produce a rapid increase in blood sugar and liver glycogen. Futhermore digestibility and caloric availability assays in the rat have indicated that dextran is highly digestible.

Enteral administration of high molecular weight "native" dextrans is described in British Patent Specification No: 830951, i.e. GB—A—830951. However, such high molecular weight dextrans generally have a low osmolarity and this may lead to constipation in a patient.

A number of liquid formulations for the enteral feeding of patients are available. These are designed either to be swallowed or to be administered to the stomach, e.g. of an unconscious patient, by means of a tube.

These liquids usually contain a balance of foodstuff and/or appropriate minerals and vitamins. However, these formulations seldom if ever contain much material which is not absorbed on the passage through the gastrointestinal tract in consequence if the liquid feed is used for a protracted period normal intestinal motility can be impaired and the patient can become severely constipated.

There is also a need for a water-soluble bulking agent, which does not produce a surge of blood sugar, and which preferably yields no, or a small number of, calories, when introduced into the gastrointestinal tract. Such a bulking agent would be of use in compositions to be administered to diabetics, the obese or others where it is desired to control the calorie intake and minimise increases in blood sugar levels after administration of food. There is still a further need for a water-soluble hydrophilic bulking agent, which is substantially unabsorbed by the small intestine, for use in the treatment of conditions of the lower bowel.

Surprisingly, and contrary to the teaching of the art, we have found that certain dextrans do not produce any large or immediate increase in blood sugar and are not absorbed to an appropriate extent by man when administered enterally. Furthermore, in contrast to other carbohydrates (e.g. glucose or dextrin), these dextrans do not produce a substantial rise in blood insulin after ingestion. We have also surprisingly found that certain dextrans can reduce the absorption of other carbohydrates, e.g. glucose. These dextrans are therefore indicated for use as an essentially non-absorbed pharmaceutical or foodstuff excipient.

According to the invention a formulation is manufactured, suitable for enteral administration to man, comprising a bulking proportion of a dextran having an average molecular weight of from 50,000 to 1,000,000.

The administration of this specific composition to control the calorie intake of a human, to provide a bulking agent for a human or of treatment of a condition of the gastrointestinal tract in a human, comprises administering enterally a composition containing a dextran of average molecular weight of from 50,000 to 1,000,000 to the patient.

The invention also provides a method of producing a formulation suitable for controlling the calorie intake of a human, for providing a bulking agent for a human or for the treatment of a condition of the gastrointestinal tract, which comprises incorporating a dextran of average molecular weight of from 50,000 to 1,000,000 into an appropriate formulation.

The compositions are indicated for use in the treatment of, or as an adjunct in the treatment of pathological conditions of the lower gastrointestinal tract such as Crohn's disease, ulcerative colitis, proctitis, coeliac disease regional ileitis or irritable bowel syndrome. The compositions are also indicated for use in controlling the blood glucose levels of diabetics and/or controlling calorie intake in those who are overweight or obese. The compositions are further indicated for pathological conditions where a bulking agent is required, e.g. to promote the flow of material through the gastrointestinal tract.

We prefer the composition to contain up to 30%, preferably up to 25%, e.g. up to 10%, w/w of the above dextran. The composition also preferably contains more than 0.5% and preferably more than 2%, w/w of the dextran.

The extran has an average molecular weight of greater than 50,000, more preferably of greater than 200,000, and especially of 250,000. The used dextran has an average molecular weight of up to 1,000,000 and 50,000 to 400,000. By dextran we mean a poly-glucose in which the glucose units are predominantly (e.g. 90% or more) linked in an alpha 1,6-configuration. Dextrans can be made by the action of the dextran-sucrase family of enzymes on sucrose. Dextransucrases are produced by various species of lactobacillae. We particularly prefer dextrans produced by *Leuconostoc mesenteroides* (and especially strain NRRL B512F). The dextrans produced by the action of the enzymes on sucrose are generally of very high molecular weight and may be partially hydrolysed and the hydrolysate fractionated to produce fractions of relatively narrow bands of lower molecular weight. We prefer to use these fractionated dextrans. These dextrans are generally available in commerce and are of the same general type as, but are usually of a higher molecular weight than, the dextrans which are currently used for intravenous administration.

The dextran may be used in any suitable form, but is preferably incorporated in a foodstuff. The foodstuff is preferably a liquid, but may also be solid, e.g. a baked prdouct such as bread or biscuits.

In liquids we have found that dextrans provide a useful thickening effect and also produce a pleasant and acceptable 'mouth feel'. The dextrans also provide a feeling of bulk in the stomach and can help prevent constipation in patients who are fed wholly or largely on liquids, or who are subject to dietary constraints because of conditions of the large intestine or bowel.

We prefer liquid formulations to contain from 0.5 to 7.0% w/w, e.g. 2.25% w/w, of the dextran.

The liquid formulations may also contain other ingredients, such as flavours, e.g. fruit flavour; acids, e.g. phosphoric, citric, maleic, fumaric or tartaric acids; buffering agents, e.g. phosphates; sugars, e.g. fructose, sorbitol or sucrose. When the composition is to be used for a diabetic or for someone obese the calorie content should be controlled, and preferably low. However, when the composition is to be used for a patient, who may be unconscious, e.g. after surgery or a stroke, special balanced nutrients may be required. Thus the compositions may contain appropiate proportions of fats, carbohydrates, proteins, minerals and vitamins. The appropriate proportions of these nutrients are well known in the art. Specificially we provide the incorporation of the dextrans of the invention into existing commercially available foodstuff formulations, particularly liquid formulations designed for administration to those who are ill or obese, e.g. those sold under the Trade Marks "Ensure" and "Osmolite".

Any sweet taste in the composition may be provided by known natural or artificial sweeteners, e.g. saccharin, aspartame or cyclamates. The presence of the dextran in the formulation can help to mask or modify the unpleasant after-taste which is present with some artificial sweeteners.

The flavours may be provided in the form of fruit syrups or concentrates or by means of artifical flavours.

The compositions may, if desired (but preferably do not), contain other permitted food additives, e.g. gums, stabilisers, flavour boosters, preservatives (e.g. sodium benzoate) and/or anti-oxidants.

When the composition is designed to be administered by tube, e.g. a nasogastric tube, it is of course desirable that it be of such a viscosity that it can be readily passed through such a tube. In general the higher the molecular weight of the dextran the greater will be both the viscosity and the osmotic effect of the product.

The compositions may be made simply by mixing the ingredients in any convenient order. The mixture may then be filled into suitable containers, e.g. 100 or 500 ml cartons or bottles, which may be pasturised or sterilised depending on the heat stability of the various components.

The dextrans have considerable hydrogen bonding ability and are heavily hydrated in the gastrointestinal tract. Thus the presence of the dextran in the compositions according to the invention can cause an increase in the water content of, and hence the general flow of material through, the gastrointestinal tract. The dextrans also have some anti-bacterial action and/or promote a favourable balance of bacteria in the gut. The compositions also help the flow of potentially toxic or allergic substances such as gluten peptides, enabling these to pass through the gut before they are absorbed.

The invention is illustrated by the following Examples.

### Example 1 (Diabetic drink)

| | |
|---|---|
| Dextran average molecular weight 500,000 | 2.5 g |
| Fructose | 5.0 g |
| Citric acid anhydrous | 0.125 g |
| Blackcurrant concentrate | 1.12 ml |
| Distilled water | to 100 ml |

### Example 2 (Nutrient formulation)

| | |
|---|---|
| Protein | 37 g/l |
| Carbohydrate (other than dextran) | 145 g/l |
| Fat | 37 g/l |
| Electrolytes, sodium, potassium and chloride | qs |
| Vitamins and minerals | qs |
| Dextran average molecular weight 500,000 | 25 g/l |

3

Example 3

A liquid preparation is as follows:

| | | |
|---|---|---|
| Dextran m/w 250,000 | 5% | w/w |
| Sod. Benzoate | 0.14% | w/w |
| Citric Acid | 0.4% | w/w |
| Orange Flavour | 0.3% | w/w |
| Sunset Yellow | 0.08% | w/w |
| Saccharin Sodium | 0.1% | w/w |

Alternative sweeteners (Thaumatin or Aspartame) and flavours may be employed.

Example 4

Four consenting adult human volunteers were, on separate occasions, given 50 g each of glucose, sucrose, "Caloreen" (a polyglucose) and a dextran of average molecular weight 500,000 by mouth. The blood sugar and insulin levels of the volunteers were followed over a period of four hours after each administration. It was found that the blood sugar and insulin levels were considerably lower after the administration of the dextran than after the administration of the other materials.

Example 5

Ten consenting adult human volunteers were, after fasting and on separate occasions, given 50 g each of glucose and "Caloreen" (a polyglucose produced by the methods described in US Patent No. i.e. US—A—3,928,135) by mouth. The volunteer's serum glucose and serum insulin levels were measured at various times. The results are shown in Tables I and II.

Fourteen consenting adult human volunteers were, after fasting, given 50 g each of, in centre A, dextran of m/w 70,000 and, in centre B, dextran of m/w 250,000. The serum glucose and serum insulin levels, which are shown in Table III, show much lower increases than those in Tables I and III.

## Table I     Mean of 10 Volunteers after 50g Glucose

| Time (Mins) | Serum Glucose levels % increase | Serum Insulin levels % increase |
|---|---|---|
| 0 | 100 | 100 |
| 30 | 168 | 710 |
| 60 | 141 | 610 |
| 90 | 120 | 580 |
| 120 | 97 | 280 |
| 150 | 94 | 210 |

Table II   Mean of 10 Volunteers after 50g Polyglucose

| Time (Mins) | Serum Glucose levels % increase | Serum Insulin levels % increase |
|---|---|---|
| 0 | 100 | 100 |
| 30 | 165 | 730 |
| 60 | 140 | 590 |
| 90 | 110 | 410 |
| 120 | 106 | 340 |
| 150 | 100 | 220 |

Table III   Mean of Two Groups of 8 & 6 Volunteers after 50g Dextran

| Time (Mins) | Serum Glucose Levels % increase | | Serum Insulin Levels levels | |
|---|---|---|---|---|
| | A | B | A | B |
| 0 | 100 | 100 | - | 100 |
| 30 | 119 | 116 | - | 205 |
| 60 | 115 | 120 | - | 205 |
| 90 | 109 | 113 | - | 125 |
| 120 | 109 | 106 | - | 125 |
| 150 | 100 | 96 | - | |
| 180 | 94 | - | | |
| 210 | 97 | - | | |
| 240 | 97 | - | | |
| 270 | 95 | - | | |
| 300 | 96 | | | |

## Example 6

In two of the volunteers in Centre A samples of serum were analysed (after removal of protein) by size exclusion chromatography. This technique is capable of determining amounts of oligosaccharides and simple sugars in the sample undergoing analysis. Table IV shows the % composition of serum carbohydrate in the two volunteers at various times after administration of the 50 g dextran, compared with the dextran starting material.

It can be seen that there is no significant increase in serum carbohydrate during the test period other than glucose and that no oligosaccharides were absorbed.

### Table IV    Serum Carbohydrate Composition (%)

### After 50g Dextran

| Polymer Degree of Polymerisation | Volunteer SA Time (Secs) | | | Volunteer GG Time (Secs) | | | Dextran Administered |
|---|---|---|---|---|---|---|---|
| | 0 | 60 | 300 | 0 | 60 | 300 | |
| 1(glucose) | 88.3 | 94.2 | 92.6 | 88.1 | 93.4 | 92.7 | 1.3 |
| 2(isomaltose)) | ) | | | ) | ) | ) | ) |
| 3 | ) | | | ) 1.0 | ) | ) | ) |
| 4 | ) | | | 1.5 | ) | ) | ) |
| 5 | ) | | | ) | ) | ) | ) |
| 6 | ) | | | ) 0.5 | ) | ) | ) |
| 7 | ) 3.8 | 1.3 | 2.1 | 0.8 | )1.3 | )1.3 | )2.7 |
| 8 | ) | | | ) | ) | ) | ) |
| 9 | ) | | | ) 2.1 | ) | ) | ) |
| 10 | ) | | | ) | ) | ) | ) |
| 11 | ) | | | ) | ) | ) | ) |
| 12 | ) | | | ) | ) | ) | ) |
| Intermediate Mol. Wt. | 1.6 | 0 | 0.6 | 1.1 | 0 | 0 | 8.1 |
| High Mol. Wt. | 6.4 | 4.5 | 4.7 | 5.0 | 5.5 | 7.1 | 87.9 |
| Conc. Mg/ml | 0.99 | 1.1 | 0.63 | 0.88 | 0.99 | 0.78 | |

## Example 7

In a further experiment dextran m/w 250,000 was incubated for two hours at 37°C with artificial gastric juice. Analysis by size exclusion chromatography failed to detect any glucose in the product after this time.

## Example 8

A human volunteer fitted with a ileostomy bag was administered 50 g of dextran orally. The subject did not have a large intestine and the action of the normal digestive enzymes could therefore be studied *in*

*vivo*. The subject was fasted overnight and at 0 time given 300 ml water containing 50 g dextran m/w 70,000 and a radio labelled polyethylene glycol (PEG). Water was given during the succeeding five hours. All ileostomy fluid was collected and analysed for dextran and PEG content and for dextran molecular weight distribution. The recoveries of dextran and PEG are shown in Table V. All dextran samples collected had the same molecular weight distribution as the starting material indicating that no hydrolysis of the dextran polymer had taken place. The recovery of the dextran matched closely that of the indigestible and non-absorbed polymer PEG showing that little, if any, absorption of dextran or dextran hydrolysis products had taken place.

**Table V    Dextran and PEG Recovery in Ileostomy Patient**

| Time | Dextran in Ileostomy Bag g | Dextran in Ileostomy Bag % Recovery | PEG in Ileostomy Bag % Recovery |
|---|---|---|---|
| 0 | - | | |
| 60 | 0.95 | 1.9 | 0 |
| 120 | 0.13 | 0.3 | 0.08 |
| 180 | 0.33 | 0.7 | 0.21 |
| 240 | 4.21 | 8.4 | 6.49 |
| 300 | 15.50 | 31.0 | 47.30 |
| | 21.10 | 42.3 | 54.08 |

## Example 9

Two volunteers were on separate occasions administered 50 g dextran m/w 70,000, 50 g glucose and 50 g dextran + 50 g glucose orally in a standard glucose tolerance test procedure. The average % increase in serum glucose levels against time are shown in Table VI for the three experiments and compared with the calculated rise from the sum of the separate glucose and dextran data. It can be seen that the administration of 50 g glucose together with 50 g dextran gives lower serum glucose levels than 50 g glucose alone and appreciably less than the calculated levels for the combined product.

**Table VI    Reduction of Glucose Absorption by Dextran**

| Time (Mins) | % Increase in Serum Glucose Levels Glucose | Dextran | Glucose + Dextran | Glucose + Dextran (calc) |
|---|---|---|---|---|
| 0 | 100 | 100 | 100 | 100 |
| 30 | 161 | 111 | 136 | 172 |
| 60 | 151 | 119 | 142 | 170 |
| 90 | 112 | 110 | 93 | 122 |
| 120 | 103 | 99 | 84 | - |
| 150 | 97 | 95 | 86 | - |
| 180 | 98 | 95 | 87 | - |

7

# EP 0 153 013 B1

**Claims**

1. The use of a dextran having an average molecular weight of from 50,000 to 1,000,000 in the manufacture of a formulation which does not increase the blood sugar or blood insulin level for the controlled calorie intake of a human or for providing a bulking agent for a human or for the treatment of a pathological condition of the gastrointestinal tract.

2. The use of a dextran according to Claim 1 characterised in that the formulation contains from 0.5 to 30% w/w of dextran.

3. The use of a dextran according to Claim 2 characterised in that the formulation is liquid and contains from 0.05 to 7.0% w/w of dextran.

4. The use of a dextran according to Claim 1 characterised in that the dextran has an average molecular weight of 50,000 to 400,000.

5. The use of a dextran according to any one of the preceding claims characterised in that the dextran has been produced by *Leuconostoc mesenteroides.*

6. The use of a dextran according to any one of the preceding claims characterised in that the dextran is incorporated in a foodstuff.

7. The use of a dextran according to Claim 6 characterised in that the foodstuff is a low calorie foodstuff.

8. The use of a dextran according to any one of the preceding claims characterised in that the formulation is a liquid suitable for administration by a nasogastric tube.


**Patentansprüche**

1. Verwendung eines Dextrans mit einem durchschnittlichen Molekulargewicht von von 50 000 bis 1 000 000 in der Herstellung einer Formulierung, die den Zucker- oder Insulinspiegel des Blutes nicht erhöht, zur kontrollierten Kalorienaufnahme eines Menschen oder zum Vorsehen eines Quellmittels für eine Menschen oder zur Behandlung eines pathologischen Zustandes des Magen-Darm-Traktes.

2. Verwendung eines Dextrans nach Anspruch 1, dadurch gekennzeichnet, daß die Formulierung von 0,5 bis 30 Gew.% des Dextrans enthält.

3. Verwendung eines Dextrans nach Anspruch 2, dadurch gekennzeichnet, daß die Formulierung flüssig ist und von 0,05 bis 7,0 Gew.% des Dextrans enthält.

4. Verwendung eines Dextrans nach Anspruch 1, dadurch gekennzeichnet, daß das Dextran ein durchschnittliches Molekulargewicht von 50 000 bis 400 000 hat.

5. Verwendung eines Dextrans nach einem der vorhergehenden Ansprüche, dadurch gekennzeichnet, daß das Dextran durch Leuconostoc mesenteroides hergestellt wurde.

6. Verwendung eines Dextrans nach einem der vorhergehenden Ansprüche, dadurch gekennzeichnet, daß das Dextran einem Nahrungsmittel zugesetzt wird.

7. Verwendung eines Dextrans nach Anspruch 6, dadurch gekennzeichnet, daß das Nahrungsmittel ein Nahrungsmittel mit wenigen Kalorien ist.

8. Verwendung eines Dextrans nach einem der vorhergehenden Ansprüche, dadurch gekennzeichnet, daß die Formulierung eine Flüssigkeit ist, die zur Verabreichung durch eine Nasen-Magen-Sonde geeignet ist.


**Revendications**

1. Utilisation d'un dextrane ayant un poids moléculaire moyen de 50 000 à 1 000 000 dans la fabrication d'une composition qui n'élève pas le taux de sucre ou d'insuline dans le sang en vue de l'absorption limitée de calories chez un être humain ou pour constituer un agent de satiéte pour un être humain ou pour le traitement d'un état pathologique du tractus gastrointestinal.

2. Utilisation d'un dextrane suivant la revendication 1, caractérisée en ce que la composition contient 0,5 à 30% p/p de dextrane.

3. Utilisation d'un dextrane suivant la revendication 2, caractérisée en ce que la composition est liquide et contient 0,05 à 7% p/p de dextrane.

4. Utilisation d'un dextrane suivant la revendication 1, caractérisée en ce que le dextrane a un poids moléculaire moyen de 50 000 à 400 000.

5. Utilisation d'un dextrane suivant l'une quelconque des revendications précédentes, caractérisée en ce que le dextrane a été produit par *Leuconostoc mesenteroides.*

6. Utilisation d'un dextrane suivant l'une quelconque des revendications précédentes, caractérisée en ce que le dextrane est incorporé à un aliment.

7. Utilisation d'un dextrane suivant la revendication 6, caractérisée en ce que l'aliment est un aliment hypocalorique.

8. Utilisation d'un dextrane suivant l'une quelconque des revendications précédentes, caractérisée en ce que la composition est un liquide propre à l'administration par un tube nasogastrique.